# EUROPEAN PATENT SPECIFICATION

(11) **EP 4 122 918 B1**
(45) Date of publication and mention of the grant of the patent: **18.11.2026**
(21) Application number: 21186503.5
(22) Date of filing: 19.07.2021
(51) Int. Cl.: C07D 213/89

(54) **PROCESS FOR THE PREPARATION OF N-HYDROXYPYRIDONE COMPOUNDS**
VERFAHREN ZUR HERSTELLUNG VON N-HYDROXYPYRIDONVERBINDUNGEN
PROCESSUS DE PRÉPARATION DE COMPOSÉS DE N-HYDROXYPYRIDONE

(43) Date of publication of application: 25.01.2023
(73) Proprietor: Clariant International Ltd, 4132 Muttenz (CH)
(72) Inventor: FISCH, Lothar, 65510 Idstein (DE); MULLER, Thierry, 9026 Ettelbruck (LU); SARRIA TORO, Juan Manuel, 65795 Hattersheim (DE)
(74) Representative: Zusammenschluss Clariant

(56) References cited:
- EP-A2- 0 158 481
- EP-A2- 0 255 689
- EP-A2- 0 710 650
- WO-A2-2008/064345
- GB-A- 1 416 397
- US-A- 3 972 888

## Description

The invention relates to a process for the preparation of N-hydroxypyridone compounds from pyrone compounds.

Preservation of cosmetic formulations and household formulations extends their shelf life and therefore provides greater value for money for consumers. Furthermore, preservatives prevent consumers from distributing microbes around their home or on themselves and hence provide health benefits. Anti-microbial actives are well-described in the art and there are many available that provide excellent performance.

Piroctone Olamine, also known as Octopirox^{®} (Clariant) and as piroctone ethanolamine, is a compound used in the treatment of fungal infections. The chemical name for Piroctone Olamine is the monoethanolamine salt of 1 hydroxy-4-methyl-6-(2,4,4-trimethylpentyl)-2(1H)-pyridone. Hence, Piroctone Olamine is the ethanolamine salt of the hydroxamic acid derivative piroctone.

Piroctone Olamine is often used in anti-dandruff shampoos as a safe alternative to the commonly used compound zinc pyrithione.

GB1440975, EP0158481, and WO2006/081969, among others, describe the use of Piroctone Olamine as an anti-dandruff agent and/or as a preservative.

Nevertheless, there is a desire for improved preservation and anti-fungal systems. In particular, there is a desire for reducing the concentration of such systems - but this requires improved efficacy to ensure that sufficient microbial growth (e.g. growth of bacteria, fungi or yeast) is inhibited. Consumers have generally become more conscious about the content of cosmetic and household products, and they desire reduced levels of 'chemicals', particularly those that may have a reputation for inhibiting the growth of microorganisms.

With the foregoing in mind, there remains a need for more efficacious preservation and anti-fungal substances, which meet present performance requirements as well as modern consumer desires and expectations.

WO2019/228988 discloses a process for forming 2-hydroxypyridine-1-oxide compounds such as piroctone from the corresponding amine salt such as piroctone olamine using an acid.

Processes for the preparation of 1-hydroxy-2-pyridones by reaction of the corresponding 2-pyrone with hydroxylamine or a hydroxylammonium compound are known. US3972888 and GB1416397 describe such a reaction in the presence of an aminopyridine or imidazole, wherein the product is precipitated from an aqueous medium using sodium hydroxide. EP0255689 discloses a similar process using an alkali metal carbonate or hydrogen carbonate as base, the 1-hydroxy-2-pyridone being isolated directly or as a salt of an organic base, for example as the ethanolamine salt (piroctone olamine). WO2008/064345 describes a further process for the preparation of such 1-hydroxy-2-pyridones. EP0710650 describes a related process additionally carried out in the presence of an organic acid or a salt thereof. Surprisingly, it has now been found that N-hydroxypyridone compounds can be prepared directly from pyrone compounds and isolated in good yields.

The present invention relates to a process for the preparation of a compound of formula (I) 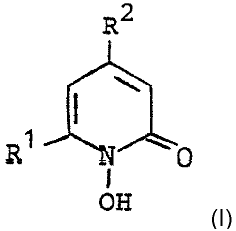 wherein
R¹ is 2,4,4-trimethylpentyl; and
R² is methyl; comprising the following steps:
   a) reacting a compound of formula (II) 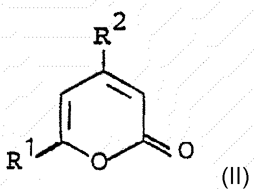 wherein
      R¹ and R² are as in the compound of formula (I);
      with hydroxylamine or a hydroxylammonium compound; and
   b) crystallizing or precipitating the compound of formula (I) from an organic solvent.

An advantage of the compound of formula (I) such as piroctone is that there is a better activity to weight ratio than for the corresponding salt such as piroctone olamine, i.e. more efficacy per molecular weight of active. For example, the olamine counter ion in piroctone olamine accounts for approximately 20% of the overall mass of piroctone olamine but does not contribute to its anti-dandruff activity.

Using the process of the present invention, piroctone can be isolated in yields that are comparable to the yields obtained in a typical synthesis of piroctone olamine.

In a typical synthesis of piroctone olamine, the corresponding pyrone is reacted with hydroxylamine or a hydroxylammonium compound, and piroctone is isolated as its ethanolamine salt (piroctone olamine). So far, the intermediate piroctone could not be isolated in reasonable yields. Using the process of the present invention, piroctone can be prepared directly from the corresponding pyrone and isolated in good yields. Advantageously, the process of the present invention provides direct access to N-hydroxypyridone compounds such as piroctone from pyrone compounds. Therefore, an additional step of converting a salt such as piroctone olamine into the free N-hydroxypyridone compound such as piroctone can be avoided, which saves time, costs, and resources. Accordingly, the process of the present invention is also interesting from an efficiency perspective as well as from a sustainability perspective.

The process of the invention is a process for the preparation of a compound of formula (I) 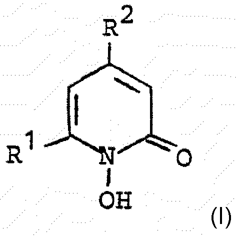 wherein
R¹ is 2,4,4-trimethylpentyl; and
R² is methyl.

This compound is also referred to herein as piroctone.

The process of the invention comprises the following steps:
a) reacting a compound of formula (II) 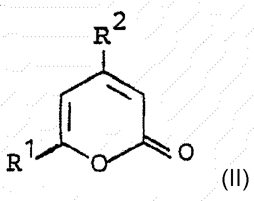 wherein
   R¹ and R² are as in the compound of formula (I);
   with hydroxylamine or a hydroxylammonium compound; and
b) crystallizing or precipitating the compound of formula (I) from an organic solvent.

### Step a)

A compound of formula (II) is reacted with hydroxylamine or a hydroxylammonium compound. In one embodiment, a compound of formula (II) is reacted with hydroxylamine. Hydroxylamine can, for example, be used in the form of an aqueous solution. Hydroxylamine is, for example, commercially available as a 50% aqueous solution. In a preferred embodiment, a compound of formula (II) is reacted with a hydroxylammonium compound.

Hydroxylammonium compounds are particularly preferred. Suitable hydroxylammonium compounds are known to a person skilled in the art.

Preferred hydroxylammonium compounds are selected from hydroxylammonium sulfate, hydroxylammonium chloride, hydroxylammonium acetate, hydroxylammonium phosphate, hydroxylammonium nitrate, hydroxylammonium perchlorate, hydroxylammonium oxalate, hydroxylammonium hydrogen sulfate, hydroxylammonium 4-methylbenzenesulfonate and hydroxylammonium bromide.

More preferred hydroxylammonium compounds are selected from hydroxylammonium sulfate, hydroxylammonium chloride and hydroxylammonium acetate.

Even more preferred hydroxylammonium compounds are selected from hydroxylammonium sulfate and hydroxylammonium chloride.

A particularly preferred hydroxylammonium compound is hydroxylammonium sulfate.

Another particularly preferred hydroxylammonium compound is hydroxylammonium chloride.

Preferably, the molar ratio of the compound of formula (II) to hydroxylamine equivalents is from 1.0:1.0 to 1.0:4.0, more preferably from 1.0:1.8 to 1.0:3.0, even more preferably from 1.0:2.0 to 1.0:2.6, particularly preferably from 1.0:2.0 to 1.0:2.4, for example 1.0:2.2.

In a preferred embodiment, the molar ratio of the compound of formula (II) to the hydroxylammonium compound is from 1.0:0.5 to 1.0:2.0, more preferably from 1.0:0.9 to 1.0:1.5, even more preferably from 1.0:1.0 to 1.0:1.3, particularly preferably from 1.0:1.0 to 1.0:1.2, for example 1.0:1.1. Such molar ratios are preferred, for example, when hydroxylammonium sulfate is used as the hydroxylammonium compound.

In another preferred embodiment, the molar ratio of the compound of formula (II) to hydroxylamine or the hydroxylammonium compound is from 1.0:1.0 to 1.0:4.0, more preferably from 1.0:1.8 to 1.0:3.0, even more preferably from 1.0:2.0 to 1.0:2.6, particularly preferably from 1.0:2.0 to 1.0:2.4, for example 1.0:2.2. Such molar ratios are preferred, for example, when hydroxylammonium chloride or hydroxylammonium acetate is used as the hydroxylammonium compound.

In preferred embodiments, the compound of formula (II) is reacted with hydroxylamine or the hydroxylammonium compound in the presence of a base.

In one embodiment, the compound of formula (II) is reacted with hydroxylamine in the presence of a base. In another embodiment, the compound of formula (II) is reacted with hydroxylamine in the absence of a base.

In a preferred embodiment, the compound of formula (II) is reacted with the hydroxylammonium compound in the presence of a base.

Suitable bases are known to a person skilled in the art.

Examples of suitable bases are metal carbonates, metal hydrogen carbonates, or metal hydroxides, such as alkali metal carbonates, alkali metal hydrogen carbonates, alkali metal hydroxides, alkaline earth metal carbonates, alkaline earth metal hydrogen carbonates, or alkaline earth metal hydroxides.

Preferred bases are selected from alkali metal carbonates, alkali metal hydrogen carbonates, alkali metal hydroxides, and mixtures thereof.

More preferred bases are selected from alkali metal carbonates, alkali metal hydrogen carbonates, and mixtures thereof.

Even more preferred bases are selected from alkali metal carbonates.

Examples of suitable bases are lithium carbonate, sodium carbonate, potassium carbonate, cesium carbonate, lithium hydrogen carbonate, sodium hydrogen carbonate, potassium hydrogen carbonate, cesium hydrogen carbonate, lithium hydroxide, sodium hydroxide, potassium hydroxide, cesium hydroxide, calcium carbonate, magnesium carbonate, barium carbonate, nickel carbonate, zirconium carbonate, calcium hydroxide, magnesium hydroxide, barium hydroxide, nickel hydroxide, or zirconium hydroxide.

Preferred bases are selected from lithium carbonate, sodium carbonate, potassium carbonate, lithium hydrogen carbonate, sodium hydrogen carbonate, potassium hydrogen carbonate, sodium hydroxide, potassium hydroxide, and mixtures thereof.

More preferred bases are selected from lithium carbonate, sodium carbonate, potassium carbonate, lithium hydrogen carbonate, sodium hydrogen carbonate, potassium hydrogen carbonate, and mixtures thereof.

Even more preferred bases are selected from lithium carbonate, sodium carbonate, potassium carbonate, and mixtures thereof. Also even more preferred bases are selected from sodium carbonate, sodium hydrogen carbonate, and mixtures thereof.

A particularly preferred base is sodium carbonate.

Preferably, the molar ratio of hydroxylamine equivalents to base equivalents is from 1.0:0.5 to 1.0:3.0, more preferably from 1.0:0.8 to 1.0:2.0, even more preferably from 1.0:1.0 to 1.0:1.5, even more preferably from 1.0:1.0 to 1.0:1.2, particularly preferably from 1.0:1.0 to 1.0:1.1, for example 1.0:1.0.

In a preferred embodiment, the molar ratio of the hydroxylammonium compound to the base is from 1.0:0.5 to 1.0:3.0, more preferably from 1.0:0.8 to 1.0:2.0, even more preferably from 1.0:1.0 to 1.0:1.5, even more preferably from 1.0:1.0 to 1.0:1.2, particularly preferably from 1.0:1.0 to 1.0:1.1, for example 1.0:1.0. Such molar ratios are preferred, for example, when hydroxylammonium sulfate is used as the hydroxylammonium compound and an alkali metal carbonate (for example sodium carbonate) is used as the base.

In preferred embodiments, the compound of formula (II) is reacted with hydroxylamine or the hydroxylammonium compound in the presence of a solvent. Suitable solvents are known to a person skilled in the art.

Preferred solvents are selected from organic solvents, water, and mixtures thereof.

Preferred organic solvents are selected from heptane, hexane, cyclohexane, methylcyclohexane, dimethylcyclohexane, toluene, benzene, methylene chloride, methanol, ethanol, isopropanol, tert-butanol, tert-amyl alcohol, dimethylformamide, dimethyl sulfoxide, tetrahydrofuran, acetonitrile, 2-aminopyridine, and mixtures thereof.

More preferred organic solvents are selected from heptane, hexane, cyclohexane, methylcyclohexane, toluene, 2-aminopyridine, and mixtures thereof.

A particularly preferred organic solvent is heptane.

In a preferred embodiment, the solvent is a mixture of an organic solvent and water.

In a more preferred embodiment, the solvent is a mixture of an organic solvent and water, wherein the organic solvent is selected from heptane, hexane, cyclohexane, methylcyclohexane, dimethylcyclohexane, toluene, benzene, methylene chloride, methanol, ethanol, isopropanol, tert-butanol, tert-amyl alcohol, dimethylformamide, dimethyl sulfoxide, tetrahydrofuran, acetonitrile, 2-aminopyridine, and mixtures thereof.

In an even more preferred embodiment, the solvent is a mixture of an organic solvent and water, wherein the organic solvent is selected from heptane, hexane, cyclohexane, methylcyclohexane, toluene, 2-aminopyridine, and mixtures thereof.

In a particularly preferred embodiment, the solvent is a mixture of an organic solvent and water, wherein the organic solvent is heptane.

Preferably, the solvent comprises at least 80 wt.-%, more preferably at least 90 wt.-%, even more preferably at least 95 wt.-%, particularly preferably at least 98 wt.-%, of an organic solvent, based on the total weight of the solvent.

In a preferred embodiment, the solvent comprises at least 80 wt.-%, more preferably at least 90 wt.-%, even more preferably at least 95 wt.-%, particularly preferably at least 98 wt.-%, of heptane, based on the total weight of the solvent.

Preferably, the compound of formula (II) is reacted with hydroxylamine or the hydroxylammonium compound at a temperature of 50 to 120 °C, more preferably 60 to 100 °C, even more preferably 70 to 90 °C, particularly preferably 80 to 90 °C.

Preferably, the compound of formula (II) is reacted with hydroxylamine or the hydroxylammonium compound for at least 4 hours, more preferably at least 8 hours, even more preferably at least 12 hours, particularly preferably at least 15 hours. For example, the compound of formula (II) is reacted with hydroxylamine or the hydroxylammonium compound for 8 to 24 hours, preferably 12 to 20 hours, particularly preferably 15 to 18 hours.

The reaction of the compound of formula (II) with hydroxylamine or the hydroxylammonium compound can be worked up in a usual manner, for example as described in Example 1.

### Step b)

The compound of formula (I) is crystallized or precipitated from an organic solvent. In a preferred embodiment, the compound of formula (I) is crystallized from an organic solvent. In another embodiment, the compound of formula (I) is precipitated from an organic solvent.

The organic solvent used in step b) is selected from heptane, hexane, cyclohexane, methylcyclohexane, dimethylcyclohexane, and mixtures thereof.

A particularly preferred organic solvent is heptane.

Preferably, the compound of formula (I) is crystallized at a temperature of 0 to 25 °C, more preferably 0 to 20 °C, even more preferably 0 to 15 °C, particularly preferably 0 to 10 °C.

Preferably, the compound of formula (I) is crystallized from a solution of the compound of formula (I) in the organic solvent, wherein the organic solvent has a temperature of 50 to 75 °C, preferably 55 to 70 °C, particularly preferably 60 to 65 °C. Preferably, the solution of the compound of formula (I) is cooled to a temperature of 0 to 25 °C, more preferably 0 to 20 °C, even more preferably 0 to 15 °C, particularly preferably 0 to 10 °C.

The crystallization of the compound of formula (I) can be carried out in a usual manner, for example as described in Example 1.

Preferably, the compound of formula (I) is precipitated at a temperature of 0 to 25 °C, more preferably 0 to 20 °C, even more preferably 0 to 15 °C, particularly preferably 0 to 10 °C.

Preferably, the compound of formula (I) is precipitated from a solution of the compound of formula (I) in the organic solvent, wherein the organic solvent has a temperature of 50 to 75 °C, preferably 55 to 70 °C, particularly preferably 60 to 65 °C. Preferably, the solution of the compound of formula (I) is cooled to a temperature of 0 to 25 °C, more preferably 0 to 20 °C, even more preferably 0 to 15 °C, particularly preferably 0 to 10 °C.

The precipitation of the compound of formula (I) can be carried out in a usual manner.

In a preferred embodiment, the same organic solvent is used in step a) and in step b). This is beneficial from a process perspective as well as from an economic and ecological perspective. In a particularly preferred embodiment, heptane is used as the organic solvent in step a) and in step b).

The invention is further illustrated by the following examples, without being limited thereby.

### Examples

As used herein, the term "Piroctone" or "piroctone" refers to a compound of formula (I) wherein R¹ is 2,4,4-trimethylpentyl; and R² is methyl.

As used herein, the term "Pyron" refers to a compound of formula (II) wherein R¹ is 2,4,4-trimethylpentyl; and R² is methyl.

### Example 1

Typical procedure for the synthesis of Piroctone from Pyron in heptane:
In a 4-neck 1 L round bottom flask equipped with a mechanical stirrer, a reflux condenser and an internal thermometer the following reagents are placed: Pyron (150.00 g), sodium carbonate (80.03 g) and hydroxylammonium sulfate (123.48 g). Under stirring, heptane (technical, 99 g) is added to the mixture followed by water (1.71 g). The mixture is then heated to its boiling point. Gas evolution starts at an internal temperature of 74 °C and finishes by 86 °C. The mixture is kept at this temperature under stirring for 16 h. After this time, the temperature of the mixture is reduced to 60-65 °C and warm water is added (60 °C, 355 g) after which the stirring is continued for 30 minutes. The biphasic mixture is then transferred to a heated separatory funnel kept at a temperature of at least 65 °C. The aqueous layer is separated (typically having a pH of 8) and a mixture of sulfuric acid and water (6 g H₂SO₄ dissolved in 12 g H₂O) is used to extract the organic layer yielding an aqueous layer (pH 5 to 6) which is separated. Finally, water (25 mL) is used to wash the organic layer, the aqueous layer is separated. The organic phase is transferred to a 1 L round bottom flask using heptane (30 g) to rinse the separatory funnel. The mixture is concentrated in a rotary evaporator to remove water azeotropically (60-65 °C). Mass after concentration: ~185 g. Heptane (30 mL) is added quickly after concentration and the mixture is left to cool down to room temperature under constant stirring. During this process crystals start to form in the mixture, which however do not interfere with the stirring. The mixture is cooled to 6 °C by placing it in an ice bath and filtered over a G3 sintered glass filter. The filter cake is washed 3 times with heptane (total 130 g). The obtained solid is further dried on a vacuum line (oil pump) overnight yielding 84.47 g of Piroctone. The mother liquor of the filtration is concentrated to dryness on the rotary evaporator. The residue is redissolved in heptane (50 mL) and left to recrystallize in a fridge (4 °C). After filtration and drying, additional 3.55 g of Piroctone are obtained. The new mother liquor is discarded.

Yield (Piroctone): 88.02 g Piroctone, corresponding to a yield of 57.9 %

Remark:
Crystallization or precipitation of piroctone gives comparable yields irrespective of whether heptane is added or not after the water has been removed azeotropically.

### Example 2

Cosmetic compositions (ingredients are given in wt%)

| **Example** | **2a** | **2b** | **2c** | **2d** | **2e** |
|---|---|---|---|---|---|
| Composition | Liquid soap | Body wash | Wet wipe lotion | Shampoo | Shampoo |
| Piroctone | 0.5 | 0.5 | 0.5 | 0.5 | 0.5 |
| SLES | 10 | - | - | - | 9 |
| Cocamido-propylbetaine | 2 | 4.5 | - | - | 2 |
| Cocamide MEA | - | - | - | - | 1 |
| Sodium Cocoyl Glutamate | - | 2 | 1 | 2 | - |
| Sodium Cocoyl Glycinate | - | 2 | 1 | 2 | - |
| Sodium Cocoyl Isethionate | - | 2 | - | - | - |
| Cocoyl Methyl Glucamide | - | 3 | 2 | - | - |
| EGDS | - | 0.5 | - | - | 0.7 |
| PEG-7 Glyceryl Cocoate | - | - | 1 | - | - |
| Cocoyl Glucoside | - | - | - | 5 | - |
| Lauryl Glucoside | - | - | - | 5 | - |
| Acrylates Copolymer | - | - | - | 2 | - |
| Glycerin | - | - | - | 1 | 2 |
| Panthenol | - | - | - | 0.2 | 0.2 |
| Dimethicone | - | - | - | - | - |
| Polyquaternium 7 (PQ-7) | - | - | - | - | 0.6 |
| Guar Hydroxypropyltrimonium Chloride | - | - | - | - | 0.3 |
| Hydrolyzed Protein | - | - | - | - | 0.1 |
| N-fatty acyl-N-methyl cyclic glucamide | 1.5 | 2 | 1.0 | 1.5 | 2 |
| NaCl | 1.5 | 1.0 | 0.5 | 0.3 | 1.5 |
| Water | QSP | QSP | QSP | QSP | QSP |
| Total | 100 | 100 | 100 | 100 | 100 |

### Example 3

Cosmetic compositions (ingredients are given in wt%)

| **Example** | **3a** | **3b** | **3c** | **3d** |
|---|---|---|---|---|
| Composition | Body Lotion | Intensive hand and body lotion | Anti-ageing night cream | Baby cream |
| Piroctone | 0.2 | 0.2 | 0.2 | 0.1 |
| Ethyhexyl Stearate | 7 | - | - | - |
| Decyl Oleate | 5 | - | - | - |
| Plantasens^{®} Natural Emulsifier HE 20 (Clariant), which is Cetearyl Glucoside (and) Sorbitan Olivate | 3 | - | - | - |
| Dimethicone | 2 | - | - | - |
| Glycerin | 3 | 3 | 7 | - |
| Xanthan Gum | 0.2 | - | - | - |
| Aristoflex^{®} AVC from Clariant (Ammonium Acryloyldimethyltaurate / VP Copolymer) | 0.5 | 1 | - | - |
| Polyglyceryl-2-Sesquiisostearate | - | 0.5 | - | 2 |
| Trilaureth-4 Phosphate | - | 2 | - | - |
| Mineral Oil (Paraffinum Liquidum) | - | 5 | - | - |
| Plantasens^{®} Refined Organic Babassu Butter from Clariant (Orbignya Oleifera Seed Oil) | - | 2 | - | - |
| Squalane | - | 2 | 12 | - |
| Macadamia Ternifolia Seed Oil (and) Crambe Abyssinica Seed Oil (and) Orbignya Oleifera Seed Oil | - | 2 | - | - |
| Silcare^{®} Silicone SEA from Clariant (Trideceth-9 PG-Amodimethicone and Trideceth-12) | - | 0.5 | - | - |
| Cetearyl Alcohol | - | 2 | - | - |
| Isopropyl Palmitate | - | 4 | - | - |
| Dow Corning^{®} 345 (Cyclopentasiloxane and Cyclohexasiloxane) | - | - | 10 | - |
| Paraffin Oil, low viscosity | - | - | 4 | 10 |
| Petrolatum | - | - | 4 | 15 |
| Cetyl Alcohol | - | - | 3 | - |
| Cutina^{®} GMS (Glyceryl Stearate) | - | - | 2.5 | - |
| PEG-40 Stearate | - | - | 3 | - |
| Cera Alba Wax | - | - | 2 | - |
| Mangifera Indica (Mango) Seed Butter | - | - | 2 | - |
| Abyssinian Oil (and) Phytosterols (and) Olea Europea (Olive) Oil Unsaponifiables | - | - | 0.5 | - |
| Sorbitan Tristearate | - | - | 0.3 | - |
| Ubiquinone | - | - | 0.05 | - |
| Aristoflex^{®} Velvet from Clariant (Polyacrylate Crosspolymer-11) | - | - | 0.3 | - |
| Hostacerin^{®} SFO from Clariant (Sunflower Seed Oil Sorbitol Esters) | - | - | - | 3.5 |
| Paracera^{®} M (Cera Microcristallina) | - | - | - | 2 |
| Beeswax | - | - | - | 1 |
| Magnesium Stearate | - | - | - | 1 |
| Talc | - | - | - | 10 |
| Zinc oxide | - | - | - | 10 |
| Allantoin (Clariant) | - | - | - | 0.3 |
| Extrapon Hamamelis | - | - | - | 2 |
| D-Panthenol | - | - | - | 2 |
| N-fatty acyl-N-methyl cyclic glucamide | 1.5 | 2 | 1.0 | 1.5 |
| Water | QSP | QSP | QSP | QSP |
| Total | 100 | 100 | 100 | 100 |

### Example 4

Cosmetic compositions (ingredients are given in wt%)

| **Example** | **4a** | **4b** | **4c** | **4d** | **4e** | **4f** |
|---|---|---|---|---|---|---|
| Composition | Soap formulation | Soap bar | Body wash | Shampoo | Wash cream | Body wash |
| Piroctone | 0.5 | 1.0 | 0.5 | 0.5 | 0.5 | 0.5 |
| Sodium Lauryl Sulfate | - | - | 10 | - | - | - |
| Ammonium Lauryl Sulfate | - | - | - | 12 | - | - |
| Sodium Lauryl Ether Sulfate (SLES) | 15 | - | 2 | - | - | - |
| Cocamido-propylbetaine | 7 | - | 2 | 3 | - | 2 |
| Cocamide MEA | - | - | 1 | - | - | 2 |
| Lauric Acid | 0.5 | 2 | - | - | - | 10 |
| Myristic Acid | 1.5 | 2 | - | - | - | 10 |
| Stearic Acid | 0.5 | 2 | - | - | 10 | - |
| Palmitic Acid | - | 2 | - | - | - | 10 |
| Potassium Tallowate | - | 2 | - | - | - | - |
| Sodium Palm Kernelate | - | 20 | - | - | - | - |
| Sodium Cocoate | - | 60 | - | - | - | - |
| Lauryl Glucoside | - | - | - | 4 | - | - |
| Sodium Cocoyl Isethionate | - | - | - | - | 14 | - |
| Glycerin | - | 5 | - | - | - | 5 |
| Cetearyl Alcohol | 1.5 | - | - | - | 2 | - |
| Dimethicone | - | - | - | - | 0.1 | - |
| Polyquaternium 7 (PQ-7) | - | - | - | 0.2 | - | - |
| Guar Hydroxypropyltrimonium Chloride | - | - | 0.3 | - | - | - |
| Hydrolyzed Protein | - | - | - | 0.1 | - | - |
| N-fatty acyl-N-methyl cyclic glucamide | 1.5 | 2 | 1.0 | 1.5 | 1 | 0.8 |
| NaCl | 1.5 | - | 0.5 | 0.3 | - | 2.5 |
| Water | QSP | QSP | QSP | QSP | QSP | QSP |
| Total | 100 | 100 | 100 | 100 | 100 | 100 |

### Example 5

Hair conditioner compositions

| **Example** | **5a** | **5b** | **5c** | **5d** | **5e** | **5f** |
|---|---|---|---|---|---|---|
| Ingredient | wt.-% | wt.-% | wt.-% | wt.-% | wt.-% | wt.-% |
| Piroctone | 0.4 | 0.5 | 0.3 | 0.4 | 0.5 | 0.6 |
| Cetearyl Alcohol | 4.0 | 4.0 | 4.0 | 4.0 | 4.0 | 4.0 |
| CTAC | 2.0 | - | - | 1.0 | 1.0 | - |
| BTAC | - | 2.0 | - | 1.0 | - | 1.0 |
| BTMS | - | - | 2.0 | - | 1.0 | 1.0 |
| Xiameter PMX-200 (dimethicone) | 0.2 | 0.2 | 0.2 | 0.2 | 0.2 | 0.2 |
| Water | Ad. 100 | Ad. 100 | Ad. 100 | Ad. 100 | Ad. 100 | Ad. 100 |

### Example 6

Hair conditioner compositions

| **Example** | **6a** | **6b** | **6c** | **6d** | **6e** | **6f** |
|---|---|---|---|---|---|---|
| Ingredient | wt.-% | wt.-% | wt.-% | wt.-% | wt.-% | wt.-% |
| Piroctone | 0.4 | 0.5 | 0.3 | 0.4 | 0.5 | 0.6 |
| Cetearyl Alcohol | 4.0 | 4.0 | 4.0 | 4.0 | 4.0 | 4.0 |
| CTAC | 0.5 | 0.5 | 0.5 | - | - | - |
| BTAC | - | - | - | 0.5 | 0.5 | 0.5 |
| Genadvance^{®} Life | 1.5 | - | - | 1.5 | - | - |
| Genadvance^{®} Repair | - | 1.5 | - | - | 1.5 | - |
| Genadvance^{®} Hydra | - | - | 1.5 | - | - | 1.5 |
| Xiameter PMX-200 (dimethicone) | 0.2 | 0.2 | 0.2 | 0.2 | 0.2 | 0.2 |
| Water | Ad. 100 | Ad. 100 | Ad. 100 | Ad. 100 | Ad. 100 | Ad. 100 |

| | | | | | | |
|---|---|---|---|---|---|---|
| Genadvance^{®} Life: Clariant, INCI = Polyquaternium-116 und Butylene Glycol Genadvance^{®} Repair: Clariant, INCI = Quaternium-98 Genadvance^{®} Hydra: Clariant, INCI = Lauryl/Myristyl Polyricinoleate & Glycerin | | | | | | |

### Example 7

Hair conditioner compositions

| **Example** | **7a** | **7b** | **7c** | **7d** | **7e** | **7f** |
|---|---|---|---|---|---|---|
| Ingredient | wt.-% | wt.-% | wt.-% | wt.-% | wt.-% | wt.-% |
| Piroctone | 0.4 | 0.5 | 0.3 | 0.4 | 0.5 | 0.6 |
| Cetearyl Alcohol | 4.0 | 4.0 | 4.0 | 4.0 | 4.0 | 4.0 |
| CTAC | 0.2 | 0.2 | 0.2 | - | - | - |
| BTAC | - | - | - | 0.2 | 0.2 | 0.2 |
| Genadvance^{®} Life | 1.8 | - | - | 1.8 | - | - |
| Genadvance^{®} Repair | - | 1.8 | - | - | 1.8 | - |
| Genadvance^{®} Hydra | - | - | 1.8 | - | - | 1.8 |
| Xiameter PMX-200 (dimethicone) | 0.2 | 0.2 | 0.2 | 0.2 | 0.2 | 0.2 |
| Water | Ad. 100 | Ad. 100 | Ad. 100 | Ad. 100 | Ad. 100 | Ad. 100 |

| | | | | | | |
|---|---|---|---|---|---|---|
| Genadvance^{®} Life: Clariant, INCI = Polyquaternium-116 und Butylene Glycol Genadvance^{®} Repair: Clariant, INCI = Quaternium-98 Genadvance^{®} Hydra: Clariant, INCI = Lauryl/Myristyl Polyricinoleate & Glycerin | | | | | | |

### Example 8

Shampoo compositions

| **Example** | **8a** | **8b** | **8c** | **8d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| sodium laureth sulfate | 8 | 8 | 8 | 8 |
| sodium lauryl sulfate | 8 | 8 | 8 | 8 |
| cocamide MIPA | 4 | 4 | 4 | 4 |
| glycerin | 3.5 | 3.5 | 3.5 | 3.5 |
| glycol distearate | 3 | 3 | 3 | 3 |
| sodium chloride | 2.5 | 2.5 | 2.5 | 2.5 |
| dimethicone | 2 | 2 | 2 | 2 |
| PPG-5-ceteth-20 | 1.5 | 1.5 | 1.5 | 1.5 |
| Hair conditioning agent | 1 | 1 | 1 | 1 |
| carbomer | 0.4 | 0.4 | 0.4 | 0.4 |
| niacinamide | 0.2 | 0.2 | 0.2 | 0.2 |
| pyridoxine HCI | 0.2 | 0.2 | 0.2 | 0.2 |
| guar hydroxypropyltrimonium chloride | 0.2 | 0.2 | 0.2 | 0.2 |
| hydrolyzed soy protein | 0.2 | 0.2 | 0.2 | 0.2 |
| methyl cocoate | 0.15 | 0.15 | 0.15 | 0.15 |
| sodium cocoate | 0.15 | 0.15 | 0.15 | 0.15 |
| citric acid | As needed, pH 4.5 - 6.5 | | | |
| sodium hydroxide | As needed, pH 4.5 - 6.5 | | | |
| Colorant | As needed | | | |

### Example 9

Shampoo compositions

| **Example** | **9a** | **9b** | **9c** | **9d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| sodium laureth sulfate | 14 | 14 | 14 | 14 |
| coco-betaine | 4 | 4 | 4 | 4 |
| glycerin | 3 | 3 | 3 | 3 |
| glycol distearate | 2.5 | 2.5 | 2.5 | 2.5 |
| dimethicone | 2 | 2 | 2 | 2 |
| Hair conditioning agent | 1.5 | 1.5 | 1.5 | 1.5 |
| niacinamide | 0.5 | 0.5 | 0.5 | 0.5 |
| coconut oil | 0.5 | 0.5 | 0.5 | 0.5 |
| sodium chloride | 0.5 | 0.5 | 0.5 | 0.5 |
| olive fruit oil | 0.4 | 0.4 | 0.4 | 0.4 |
| PPG-5-ceteth-20 | 0.3 | 0.3 | 0.3 | 0.3 |
| trideceth-6 | 0.25 | 0.25 | 0.25 | 0.25 |
| polyquaternium-6 | 0.25 | 0.25 | 0.25 | 0.25 |
| amodimethicone | 0.2 | 0.2 | 0.2 | 0.2 |
| carbomer | 0.2 | 0.2 | 0.2 | 0.2 |
| cetrimonium chloride | 0.15 | 0.15 | 0.15 | 0.15 |
| citric acid | As needed, pH 4.5 - 6.5 | | | |
| sodium hydroxide | As needed, pH 4.5 - 6.5 | | | |
| Colorant | As needed | | | |

### Example 10

Shampoo compositions

| **Example** | **10a** | **10b** | **10c** | **10d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| ammonium lauryl sulfate | 16 | 16 | 16 | 16 |
| cocoamidopropyl betaine | 5 | 5 | 5 | 5 |
| sodium chloride | 2.5 | 2.5 | 2.5 | 2.5 |
| niacinamide | 2 | 2 | 2 | 2 |
| Hair conditioning agent | 1 | 1 | 1 | 1 |
| hydroxypropyl guar hydroxypropyltrimonium chloride | 0.8 | 0.8 | 0.8 | 0.8 |
| propylene glycol | 0.1 | 0.1 | 0.1 | 0.1 |
| citric acid | As needed, pH 4.5 - 6.5 | | | |
| sodium hydroxide | As needed, pH 4.5 - 6.5 | | | |
| Colorant | As needed | | | |

### Example 11

Shampoo compositions

| **Example** | **11a** | **11b** | **11c** | **11d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| sodium laureth sulfate | 16 | 16 | 16 | 16 |
| glycol distearate | 4 | 4 | 4 | 4 |
| coco-betaine | 2.5 | 2.5 | 2.5 | 2.5 |
| glycerin | 2 | 2 | 2 | 2 |
| sodium chloride | 1.5 | 1.5 | 1.5 | 1.5 |
| dimethicone | 1.5 | 1.5 | 1.5 | 1.5 |
| Hair conditioning agent | 1.5 | 1.5 | 1.5 | 1.5 |
| niacinamide | 1 | 1 | 1 | 1 |
| guar hydroxypropyltrimonium chloride | 0.8 | 0.8 | 0.8 | 0.8 |
| cocamide MIPA | 0.5 | 0.5 | 0.5 | 0.5 |
| PPG-5-ceteth-20 | 0.2 | 0.2 | 0.2 | 0.2 |
| fumaric acid | 0.15 | 0.15 | 0.15 | 0.15 |
| carbomer | 0.15 | 0.15 | 0.15 | 0.15 |
| pyridoxine HCI | 0.1 | 0.1 | 0.1 | 0.1 |
| citric acid | As needed, pH 4.5 - 6.5 | | | |
| sodium hydroxide | As needed, pH 4.5 - 6.5 | | | |
| Colorant | As needed | | | |

### Example 12

Shampoo compositions

| **Example** | **12a** | **12b** | **12c** | **12d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| sodium laureth sulfate | 16 | 16 | 16 | 16 |
| coco-betaine | 4 | 4 | 4 | 4 |
| niacinamide | 2 | 2 | 2 | 2 |
| Ribes nigrum oil | 1.5 | 1.5 | 1.5 | 1.5 |
| cocamide MIPA | 1.5 | 1.5 | 1.5 | 1.5 |
| sodium chloride | 1.5 | 1.5 | 1.5 | 1.5 |
| Hair conditioning agent | 1.2 | 1.2 | 1.2 | 1.2 |
| sodium cocoate | 0.8 | 0.8 | 0.8 | 0.8 |
| cocamide MIPA | 0.5 | 0.5 | 0.5 | 0.5 |
| Olea europaea oil (olive) fruit oil | 0.3 | 0.3 | 0.3 | 0.3 |
| PEG-55 propylene glycol oleate | 0.3 | 0.3 | 0.3 | 0.3 |
| PEG-60 hydrogenated castor oil | 0.3 | 0.3 | 0.3 | 0.3 |
| polyquaternium-7 | 0.2 | 0.2 | 0.2 | 0.2 |
| amodimethicone | 0.15 | 0.15 | 0.15 | 0.15 |
| propylene glycol | 0.1 | 0.1 | 0.1 | 0.1 |
| Butyrospermum parkii butter | 0.1 | 0.1 | 0.1 | 0.1 |
| laureth-5 carboxylic acid | 0.1 | 0.1 | 0.1 | 0.1 |
| citric acid | As needed, pH 4.5 - 6.5 | | | |
| sodium hydroxide | As needed, pH 4.5 - 6.5 | | | |
| Colorant | As needed | | | |

### Example 13

Shampoo compositions

| **Example** | **13a** | **13b** | **13c** | **13d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| sodium laureth sulfate | 10 | 10 | 10 | 10 |
| disodium cocamphodiacetate | 5 | 5 | 5 | 5 |
| glycerin | 3 | 3 | 3 | 3 |
| glycol distearate | 2 | 2 | 2 | 2 |
| sodium chloride | 1.5 | 1.5 | 1.5 | 1.5 |
| Hair conditioning agent | 1.5 | 1.5 | 1.5 | 1.5 |
| cocamide MEA | 1 | 1 | 1 | 1 |
| polyquaternium-10 | 1 | 1 | 1 | 1 |
| carbomer | 0.3 | 0.3 | 0.3 | 0.3 |
| citric acid | As needed, pH 4.5 - 6.5 | | | |
| sodium hydroxide | As needed, pH 4.5 - 6.5 | | | |
| Colorant | As needed | | | |

### Example 14

Shampoo compositions

| **Example** | **14a** | **14b** | **14c** | **14d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| sodium laureth sulfate | 12 | 12 | 12 | 12 |
| coco-betaine | 3 | 3 | 3 | 3 |
| cocamide MIPA | 2 | 2 | 2 | 2 |
| sodium chloride | 1.8 | 1.8 | 1.8 | 1.8 |
| Olea europaea (olive) fruit oil | 0.5 | 0.5 | 0.5 | 0.5 |
| Hair conditioning agent | 0.5 | 0.5 | 0.5 | 0.5 |
| PPG-5-ceteth-20 | 0.4 | 0.4 | 0.4 | 0.4 |
| PEG-55 propylene glycol oleate | 0.4 | 0.4 | 0.4 | 0.4 |
| PEG-60 hydrogenated castor oil | 0.25 | 0.25 | 0.25 | 0.25 |
| polyquaternium-10 | 0.25 | 0.25 | 0.25 | 0.25 |
| amodimethicone | 0.15 | 0.15 | 0.15 | 0.15 |
| propylene glycol | 0.15 | 0.15 | 0.15 | 0.15 |
| laureth-5 | 0.1 | 0.1 | 0.1 | 0.1 |
| citric acid | As needed, pH 4.5 - 6.5 | | | |
| sodium hydroxide | As needed, pH 4.5 - 6.5 | | | |
| Colorant | As needed | | | |

### Example 15

Shampoo compositions

| **Example** | **15a** | **15b** | **15c** | **15d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| ammonium lauryl sulfate | 15 | 15 | 15 | 15 |
| cocamidopropyl betaine | 3 | 3 | 3 | 3 |
| sodium chloride | 2 | 2 | 2 | 2 |
| propylene glycol | 1.8 | 1.8 | 1.8 | 1.8 |
| hydroxypropyl guar hydroxypropyltrimonium chloride | 1.5 | 1.5 | 1.5 | 1.5 |
| Hair conditioning agent | 1 | 1 | 1 | 1 |
| citric acid | As needed, pH 4.5 - 6.5 | | | |
| sodium hydroxide | As needed, pH 4.5 - 6.5 | | | |
| Colorant | As needed | | | |

### Example 16

Shampoo compositions

| **Example** | **16a** | **16b** | **16c** | **16d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| sodium cocoyl isethionate | 4 | 4 | 4 | 4 |
| sodium lauryl sulfoacetate | 3 | 3 | 3 | 3 |
| disodium laureth sulfosuccinate | 2.5 | 2.5 | 2.5 | 2.5 |
| cocamidopropyl betaine | 2.5 | 2.5 | 2.5 | 2.5 |
| sodium lauroyl sarcosinate | 2.5 | 2.5 | 2.5 | 2.5 |
| glycol distearate | 2 | 2 | 2 | 2 |
| glycereth-26 | 1.8 | 1.8 | 1.8 | 1.8 |
| decyl glucoside | 1.5 | 1.5 | 1.5 | 1.5 |
| hydrogenated coconut acid | 1.4 | 1.4 | 1.4 | 1.4 |
| PPG-5-ceteth-20 | 1.3 | 1.3 | 1.3 | 1.3 |
| divinyldimethicone/dimethicone copolymer | 1.2 | 1.2 | 1.2 | 1.2 |
| sodium chloride | 1.1 | 1.1 | 1.1 | 1.1 |
| amodimethicone | 1 | 1 | 1 | 1 |
| Hair conditioning agent | 1 | 1 | 1 | 1 |
| polyquaternium-7 | 1 | 1 | 1 | 1 |
| polyquaternium-10 | 0.7 | 0.7 | 0.7 | 0.7 |
| sodium isethionate | 0.5 | 0.5 | 0.5 | 0.5 |
| PEG-55 propylene glycol oleate | 0.4 | 0.4 | 0.4 | 0.4 |
| propylene glycol | 0.4 | 0.4 | 0.4 | 0.4 |
| carbomer | 0.4 | 0.4 | 0.4 | 0.4 |
| C11-15 pareth-7 | 0.3 | 0.3 | 0.3 | 0.3 |
| glycerin | 0.3 | 0.3 | 0.3 | 0.3 |
| trideceth-12 | 0.25 | 0.25 | 0.25 | 0.25 |
| laureth-9 | 0.2 | 0.2 | 0.2 | 0.2 |
| hydroxypropyltrimonium hydrolyzed wheat protein | 0.1 | 0.1 | 0.1 | 0.1 |
| citric acid | As needed, pH 4.5 - 6.5 | | | |
| sodium hydroxide | As needed, pH 4.5 - 6.5 | | | |
| Colorant | As needed | | | |

### Example 17

Shampoo compositions

| **Example** | **17a** | **17b** | **17c** | **17d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| sodium methyl cocoyl taurate | 5 | 5 | 5 | 5 |
| laureth-5 carboxylic acid | 4 | 4 | 4 | 4 |
| sodium chloride | 2 | 2 | 2 | 2 |
| cocamidopropyl betaine | 2 | 2 | 2 | 2 |
| glycerin | 1.8 | 1.8 | 1.8 | 1.8 |
| glycol distearate | 1.5 | 1.5 | 1.5 | 1.5 |
| Hair conditioning agent | 1.5 | 1.5 | 1.5 | 1.5 |
| polyquaternium-10 | 1 | 1 | 1 | 1 |
| PPG-5 ceteth-20 | 0.5 | 0.5 | 0.5 | 0.5 |
| sodium lauroyl glutamate | 0.4 | 0.4 | 0.4 | 0.4 |
| propylene glycol | 0.2 | 0.2 | 0.2 | 0.2 |
| PEG-55 propylene glycol oleate | 0.15 | 0.15 | 0.15 | 0.15 |
| Argania spinosa kernel oil | 0.1 | 0.1 | 0.1 | 0.1 |
| citric acid, lactic acid | As needed, pH 4.5 - 6.5 | | | |
| sodium hydroxide | As needed, pH 4.5 - 6.5 | | | |
| Colorant | As needed | | | |

### Example 18

Shampoo compositions

| **Example** | **18a** | **18b** | **18c** | **18d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| sodium laureth sulfate | 10 | 10 | 10 | 10 |
| disodium cocoamphodiacetate | 4 | 4 | 4 | 4 |
| glycol distearate | 2 | 2 | 2 | 2 |
| sodium chloride | 1.5 | 1.5 | 1.5 | 1.5 |
| Hair conditioning agent | 1.3 | 1.3 | 1.3 | 1.3 |
| carbomer | 0.4 | 0.4 | 0.4 | 0.4 |
| diaminopyrimidine oxide | 0.3 | 0.3 | 0.3 | 0.3 |
| disodium ricinoleamido MEA sulfosuccinate | 0.3 | 0.3 | 0.3 | 0.3 |
| propylene glycol | 0.2 | 0.2 | 0.2 | 0.2 |
| panthenol | 0.1 | 0.1 | 0.1 | 0.1 |
| polyquaternium-10 | 0.1 | 0.1 | 0.1 | 0.1 |
| citric acid | As needed, pH 4.5 - 6.5 | | | |
| sodium hydroxide, ammonium hydroxide | As needed, pH 4.5 - 6.5 | | | |
| Colorant | As needed | | | |

### Example 19

Shampoo compositions

| **Example** | **19a** | **19b** | **19c** | **19d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| sodium cocoyl isethionate | 4.5 | 4.5 | 4.5 | 4.5 |
| sodium lauryl sulfoacetate | 3 | 3 | 3 | 3 |
| disodium laureth sulfosuccinate | 2.5 | 2.5 | 2.5 | 2.5 |
| cocamidopropyl betaine | 2.5 | 2.5 | 2.5 | 2.5 |
| sodium lauroyl sarcosinate | 2.3 | 2.3 | 2.3 | 2.3 |
| glycol distearate | 2 | 2 | 2 | 2 |
| glycereth-26 | 1.8 | 1.8 | 1.8 | 1.8 |
| decyl glucoside | 1.7 | 1.7 | 1.7 | 1.7 |
| hydrogenated coconut acid | 1.5 | 1.5 | 1.5 | 1.5 |
| PPG-5-ceteth-20 | 1.5 | 1.5 | 1.5 | 1.5 |
| PEG-55 propylene glycol oleate | 1.5 | 1.5 | 1.5 | 1.5 |
| propylene glycol | 1.5 | 1.5 | 1.5 | 1.5 |
| sodium chloride | 1.5 | 1.5 | 1.5 | 1.5 |
| divinyldimethicone/dimethicone copolymer | 1 | 1 | 1 | 1 |
| polyquaternium-7 | 1 | 1 | 1 | 1 |
| amodimethicone | 1 | 1 | 1 | 1 |
| Hair conditioning agent | 1 | 1 | 1 | 1 |
| polyquaternium-10 | 0.8 | 0.8 | 0.8 | 0.8 |
| sodium isethionate | 0.6 | 0.6 | 0.6 | 0.6 |
| carbomer | 0.6 | 0.6 | 0.6 | 0.6 |
| C11-15 pareth-7 | 0.4 | 0.4 | 0.4 | 0.4 |
| glycerin | 0.3 | 0.3 | 0.3 | 0.3 |
| laureth-9 | 0.2 | 0.2 | 0.2 | 0.2 |
| citric acid | As needed, pH 4.5 - 6.5 | | | |
| sodium hydroxide | As needed, pH 4.5 - 6.5 | | | |
| Colorant | As needed | | | |

### Example 20

Shampoo compositions

| **Example** | **20a** | **20b** | **20c** | **20d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| cocamidopropyl betaine | 5 | 5 | 5 | 5 |
| sodium lauryl sulfoacetate | 4 | 4 | 4 | 4 |
| disodium laureth sulfosuccinate | 3.5 | 3.5 | 3.5 | 3.5 |
| sodium lauroyl sarcosinate | 3 | 3 | 3 | 3 |
| glycol distearate | 2 | 2 | 2 | 2 |
| sodium chloride | 1.7 | 1.7 | 1.7 | 1.7 |
| decyl glucoside | 1.5 | 1.5 | 1.5 | 1.5 |
| Hair conditioning agent | 1.5 | 1.5 | 1.5 | 1.5 |
| polyquaternium-10 | 1 | 1 | 1 | 1 |
| PPG-5-ceteth-20 | 1 | 1 | 1 | 1 |
| coco-betaine | 0.8 | 0.8 | 0.8 | 0.8 |
| PEG-55 propylene glycol oleate | 0.8 | 0.8 | 0.8 | 0.8 |
| propylene glycol | 0.5 | 0.5 | 0.5 | 0.5 |
| carbomer | 0.4 | 0.4 | 0.4 | 0.4 |
| citric acid | As needed, pH 4.5 - 6.5 | | | |
| sodium hydroxide | As needed, pH 4.5 - 6.5 | | | |
| Colorant | As needed | | | |

### Example 21

Shampoo compositions

| **Example** | **21a** | **21b** | **21c** | **21d** |
|---|---|---|---|---|
| Ingredient | wt% | wt% | wt% | wt% |
| Aqua | To 100 | To 100 | To 100 | To 100 |
| Piroctone | 0.1 | 0.3 | 0.7 | 1.0 |
| sodium laureth sulfate | 15 | 15 | 15 | 15 |
| coco-betaine | 3 | 3 | 3 | 3 |
| glycol distearate | 2.5 | 2.5 | 2.5 | 2.5 |
| sodium chloride | 2 | 2 | 2 | 2 |
| PPG-5-ceteth-20 | 1.8 | 1.8 | 1.8 | 1.8 |
| Hair conditioning agent | 1.5 | 1.5 | 1.5 | 1.5 |
| octyldodecanol | 1 | 1 | 1 | 1 |
| carbomer | 1 | 1 | 1 | 1 |
| guar hydroxypropyltrimonium chloride | 1 | 1 | 1 | 1 |
| sodium hyaluronate | 0.4 | 0.4 | 0.4 | 0.4 |
| citric acid | As needed, pH 4.5 - 6.5 | | | |
| sodium hydroxide | As needed, pH 4.5 - 6.5 | | | |
| Colorant | As needed | | | |

### Example 22

Bar shampoos

| **Example** | **22a** | **22b** | **22c** | **22d** |
|---|---|---|---|---|
| Ingredient | wt.-% | wt.-% | wt.-% | wt.-% |
| Piroctone | 0.3 | 0.5 | 1.0 | 0.1 |
| Sodium Methyl Cocoyl Taurate | 6 | 6 | 6 | 6 |
| Sodium Cocoyl Isethionate | 60.5 | 60.5 | 60.5 | 60.5 |
| Cocamidopropyl Betaine | 3 | 3 | 3 | 3 |
| PEG-180 | 25 | 25 | 25 | 25 |
| Quaternium-98 (Genadvance Repair) | 4 | 4 | 4 | 4 |
| Parfum | 0.5 | 0.5 | 0.5 | 0.5 |
| Preservative | As needed | | | |

## Claims

1. A process for the preparation of a compound of formula (I) 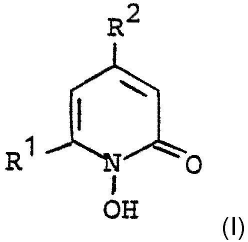 wherein
R¹ is 2,4,4-trimethylpentyl; and
R² is methyl;
comprising the following steps:
a) reacting a compound of formula (II) 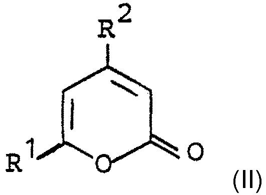 wherein
R¹ and R² are as in the compound of formula (I);
with hydroxylamine or a hydroxylammonium compound; and
b) crystallizing or precipitating the compound of formula (I) from an organic solvent, wherein the organic solvent used in step b) is selected from heptane, hexane, cyclohexane, methylcyclohexane, dimethylcyclohexane, and mixtures thereof.

2. The process according to claim 1, wherein the compound of formula (II) is reacted with the hydroxylammonium compound.

3. The process according to claim 1 or 2, wherein the hydroxylammonium compound is selected from hydroxylammonium sulfate, hydroxylammonium chloride and hydroxylammonium acetate.

4. The process according to any of claims 1 to 3, wherein the compound of formula (II) is reacted with hydroxylamine or the hydroxylammonium compound in the presence of a base.

5. The process according to claim 4, wherein the base is selected from alkali metal carbonates, alkali metal hydrogen carbonates, alkali metal hydroxides, and mixtures thereof.

6. The process according to any of claims 1 to 5, wherein the compound of formula (II) is reacted with hydroxylamine or the hydroxylammonium compound in the presence of a solvent.

7. The process according to claim 6, wherein the solvent comprises at least 80 wt.-%, preferably at least 90 wt.-%, of an organic solvent, based on the total weight of the solvent.

8. The process according to claim 7, wherein the organic solvent is selected from heptane, hexane, cyclohexane, methylcyclohexane, toluene, 2-aminopyridine, and mixtures thereof, preferably is heptane.

9. The process according to any of claims 1 to 8, wherein the compound of formula (II) is reacted with hydroxylamine or the hydroxylammonium compound at a temperature of 60 to 100 °C, preferably 70 to 90 °C.

10. The process according to any of claims 1 to 9, wherein in step b) the compound of formula (I) is crystallized from the organic solvent.

11. The process according to any of claims 1 to 10, wherein the organic solvent used in step b) is selected from heptane, hexane, cyclohexane, and mixtures thereof.

12. The process according to any of claims 1 to 11, wherein the organic solvent used in step b) is heptane.

13. The process according to any of claims 1 to 12, wherein in step b) the compound of formula (I) is crystallized or precipitated at a temperature of 0 to 25 °C, preferably 0 to 20 °C.

## Patentansprüche

1. Verfahren zur Herstellung einer Verbindung der Formel (I) 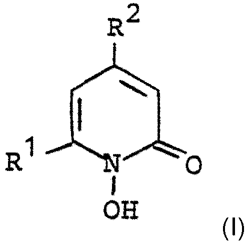 wobei
R¹ 2,4,4-Trimethylpentyl ist; und
R² Methyl ist;
umfassend die folgenden Schritte:
a) Umsetzen einer Verbindung der Formel (II) 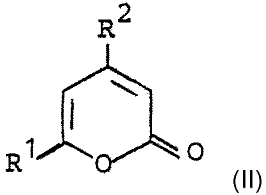 wobei
R¹ und R² die in der Verbindung der Formel (I) angegebenen Bedeutungen haben;
mit Hydroxylamin oder einer Hydroxylammoniumverbindung; und
b) Kristallisieren oder Ausfällen der Verbindung der Formel (I) aus einem organischen Lösungsmittel, wobei das in Schritt b) verwendete organische Lösungsmittel aus Heptan, Hexan, Cyclohexan, Methylcyclohexan, Dimethylcyclohexan und Mischungen davon ausgewählt ist.

2. Verfahren nach Anspruch 1, wobei die Verbindung der Formel (II) mit der Hydroxylammoniumverbindung umgesetzt wird.

3. Verfahren nach Anspruch 1 oder 2, wobei die Hydroxylammoniumverbindung aus Hydroxylammoniumsulfat, Hydroxylammoniumchlorid und Hydroxylammoniumacetat ausgewählt ist.

4. Verfahren nach einem der Ansprüche 1 bis 3, wobei die Verbindung der Formel (II) mit Hydroxylamin oder der Hydroxylammoniumverbindung in Gegenwart einer Base umgesetzt wird.

5. Verfahren nach Anspruch 4, wobei die Base aus Alkalimetallcarbonaten, Alkalimetallhydrogencarbonaten, Alkalimetallhydroxiden und Mischungen davon ausgewählt ist.

6. Verfahren nach einem der Ansprüche 1 bis 5, wobei die Verbindung der Formel (II) mit Hydroxylamin oder der Hydroxylammoniumverbindung in Gegenwart eines Lösungsmittels umgesetzt wird.

7. Verfahren nach Anspruch 6, wobei das Lösungsmittel mindestens 80 Gew.-%, vorzugsweise mindestens 90 Gew.-%, eines organischen Lösungsmittels, bezogen auf das Gesamtgewicht des Lösungsmittels, umfasst.

8. Verfahren nach Anspruch 7, wobei das organische Lösungsmittel ausgewählt ist aus Heptan, Hexan, Cyclohexan, Methylcyclohexan, Toluol, 2-Aminopyridin und Mischungen davon, vorzugsweise Heptan ist.

9. Verfahren nach einem der Ansprüche 1 bis 8, wobei die Verbindung der Formel (II) mit Hydroxylamin oder der Hydroxylammoniumverbindung bei einer Temperatur von 60 bis 100 °C, vorzugsweise 70 bis 90 °C, umgesetzt wird.

10. Verfahren nach einem der Ansprüche 1 bis 9, wobei in Schritt b) die Verbindung der Formel (I) aus dem organischen Lösungsmittel kristallisiert wird.

11. Verfahren nach einem der Ansprüche 1 bis 10, wobei das in Schritt b) verwendete organische Lösungsmittel aus Heptan, Hexan, Cyclohexan und Mischungen davon ausgewählt ist.

12. Verfahren nach einem der Ansprüche 1 bis 11, wobei das in Schritt b) verwendete organische Lösungsmittel Heptan ist.

13. Verfahren nach einem der Ansprüche 1 bis 12, wobei in Schritt b) die Verbindung der Formel (I) bei einer Temperatur von 0 bis 25 °C, vorzugsweise 0 bis 20 °C, kristallisiert oder ausgefällt wird.

## Revendications

1. Procédé de préparation d'un composé de formule (I) 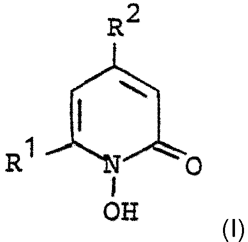 dans laquelle
R¹ représente un groupe 2,4,4-triméthylpentyle ; et
R² représente un groupe méthyle ;
comprenant les étapes suivantes :
a) la réaction d'un composé de formule (II) 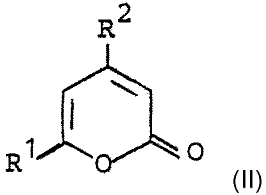 dans laquelle
R¹ et R² sont tels que définis dans le composé de formule (I) ;
avec de l'hydroxylamine ou un composé d'hydroxylammonium ; et
b) la cristallisation ou la précipitation du composé de formule (I) à partir d'un solvant organique, le solvant organique utilisé à l'étape b) étant choisi parmi l'heptane, l'hexane, le cyclohexane, le méthylcyclohexane, le diméthylcyclohexane et leurs mélanges.

2. Procédé selon la revendication 1, dans lequel le composé de formule (II) est mis à réagir avec le composé d'hydroxylammonium.

3. Procédé selon la revendication 1 ou 2, dans lequel le composé d'hydroxylammonium est choisi parmi le sulfate d'hydroxylammonium, le chlorure d'hydroxylammonium et l'acétate d'hydroxylammonium.

4. Procédé selon l'une quelconque des revendications 1 à 3, dans lequel le composé de formule (II) est mis à réagir avec de l'hydroxylamine ou le composé d'hydroxylammonium en présence d'une base.

5. Procédé selon la revendication 4, dans lequel la base est choisie parmi les carbonates de métaux alcalins, les hydrogénocarbonates de métaux alcalins, les hydroxydes de métaux alcalins et leurs mélanges.

6. Procédé selon l'une quelconque des revendications 1 à 5, dans lequel le composé de formule (II) est mis à réagir avec de l'hydroxylamine ou le composé d'hydroxylammonium en présence d'un solvant.

7. Procédé selon la revendication 6, dans lequel le solvant comprend au moins 80 % en poids, de préférence au moins 90 % en poids, d'un solvant organique, par rapport au poids total du solvant.

8. Procédé selon la revendication 7, dans lequel le solvant organique est choisi parmi l'heptane, l'hexane, le cyclohexane, le méthylcyclohexane, le toluène, la 2-aminopyridine et leurs mélanges, de préférence l'heptane.

9. Procédé selon l'une quelconque des revendications 1 à 8, dans lequel le composé de formule (II) est mis à réagir avec de l'hydroxylamine ou le composé d'hydroxylammonium à une température comprise entre 60 et 100 °C, de préférence entre 70 et 90 °C.

10. Procédé selon l'une quelconque des revendications 1 à 9, dans lequel, à l'étape b), le composé de formule (I) est cristallisé à partir du solvant organique.

11. Procédé selon l'une quelconque des revendications 1 à 10, dans lequel le solvant organique utilisé à l'étape b) est choisi parmi l'heptane, l'hexane, le cyclohexane et leurs mélanges.

12. Procédé selon l'une quelconque des revendications 1 à 11, dans lequel le solvant organique utilisé à l'étape b) est l'heptane.

13. Procédé selon l'une quelconque des revendications 1 à 12, dans lequel, à l'étape b), le composé de formule (I) est cristallisé ou précipité à une température comprise entre 0 et 25 °C, de préférence entre 0 et 20 °C.
